# EUROPÄISCHE PATENTSCHRIFT

(11) **EP 0 656 889 B1**
(45) Veröffentlichungstag und Bekanntmachung des Hinweises auf die Patenterteilung: **27.05.1998**
(21) Anmeldenummer: 93918946.0
(22) Anmeldetag: 31.08.1993
(51) Int. Cl.: C07C 405/00, A61K 31/557

(54) **9-CHLOR-PROSTAGLANDIN-ESTER UND -AMIDE UND IHRE VERWENDUNG FÜR DIE HERSTELLUNG VON ARZNEIMITTELN**
9-CHLOROPROSTAGLANDIN ESTERS AND AMIDES AND THEIR USE IN THE PREPARATION OF DRUGS
ESTERS ET AMIDES DE 9-CHLORE-PROSTAGLANDINE ET LEUR UTILISATION POUR PREPARER DES MEDICAMENTS

(30) Priorität: 31.08.1992 DE 4229051; 31.08.1992 DE 4229048; 31.08.1992 DE 4229050
(43) Veröffentlichungstag der Anmeldung: 14.06.1995
(73) Patentinhaber: SCHERING AKTIENGESELLSCHAFT, 13353 Berlin (DE)
(72) Erfinder: BUCHMANN, Bernd, D-10557 Berlin (DE); SKUBALLA, Werner, D-13465 Berlin (DE); EKERDT, Roland, D-13469 Berlin (DE); McDONALD, Fiona, D-14055 Berlin (DE); THIERAUCH, Karl-Heinz, D-14169 Berlin (DE)
(86) Internationale Anmeldenummer: DE9300809
(87) Internationale Veröffentlichungsnummer: WO9405631

(56) Entgegenhaltungen:
- EP-A- 0 030 377
- EP-A- 0 299 914
- EP-A- 0 447 015
- WO-A-86/04504
- WO-A-86/05488
- WO-A-92/08697

## Beschreibung

Gegenstand der vorliegenden Erfindung sind Ester und Amide von 9-Chlor-prostaglandinanaloga, Verfahren zu ihrer Herstellung, ihre Verwendung als Arzneimittel und diese Ester oder Amide enthaltende pharmazeutische Präparate.

Aus dem umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch auf Schwierigkeiten. Die meisten natürlichen Prostaglandine besitzen eine für therapeutische Zwecke zu kurze Wirkdauer, da sie zu rasch durch verschiedene enzymatische Prozesse abgebaut werden. Alle Strukturveränderungen haben daher das Ziel, sowohl die Wirkdauer als auch die Selektivität der Wirkung zu steigern.

9-Chlor-prostaglandinanaloga sind pharmakologisch und medizinisch wertvolle Wirkstoffe, deren Herstellung und Anwendung z.B. zur Zytoprotektion, Ulkusheilung, Hemmung der Magensäuresekretion, Luteolyse, Blutdrucksenkung, oder Plättchenaggregationshemmung in EP 299 914 und WO 86/05488 beschrieben sind. Diese Substanzen besitzen gegenüber den entsprechenden natürlichen Prostaglandinen bei ähnlichem Wirkungsspektrum eine wesentlich verbesserte Spezifität und Wirkung.

WO-A- 92/08697 bezieht sich auf 9-Halogen-MS-hydroxy-prostan-derivate, die für die Glaukom-Behandlung geeignet sind.

Aus EP 299 914 sind 9-Chlor-3-oxa-prostaglandin-Derivate der allgemeinen Formel 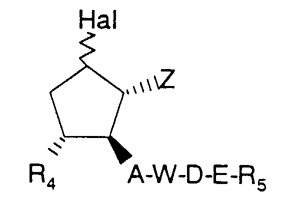 worin
- Z: die Reste 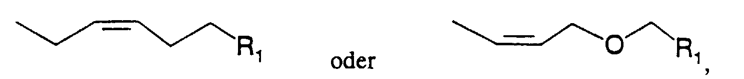
- Hal: ein α- oder β-ständiges Chlor- oder Fluoratom,
- R₁: den Rest CH₂OH oder COOR₂ mit R₂ in der Bedeutung eines Wasserstoffatoms, eines Alkyl- oder Cycloalkyl-, Aryl- oder heterocyclischen Restes oder R₁ den Rest CONHR₃ mit R₃ in der Bedeutung eines Säurerestes oder des Restes R₂ und
- A: eine -CH₂-CH₂-, eine trans -CH=CH- oder eine -C≡C-Gruppe
- W: eine freie oder funktionell abgewandelte Hydroxymethylengruppe oder eine freie oder funktionell abgewandelte 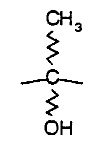 Gruppe, wobei die jeweiligen OH-Gruppen α- oder β-ständig sein können,
- D und E: gemeinsam eine direkte Bindung oder
- D: eine geradkettige mit 1-10, eine verzweigtkettige mit 2-10 oder eine ringförmige Alkylengruppe mit 3-10 C-Atomen, die gegebenenfalls durch Fluoratome substituiert sein können und
- E: ein Sauerstoff- oder Schwefelatom, eine direkte Bindung, eine -C≡C- Bindung oder eine -CR₆=CR₇-Gruppe darstellt, wobei R₆ und R₇ sich unterscheiden und ein Wasserstoffatom, ein Chloratom oder eine C₁-C₄-Alkylgruppe bedeuten,
- R₄: eine freie oder funktionell abgewandelte Hydroxygruppe,
- R₅: ein Wasserstoffatom, eine Alkyl-, eine Halogen-substituierte Alkyl-, eine Cycloalkyl-, eine gegebenenfalls substituierte Aryl- oder eine heterocyclische Gruppe, und falls R₂ die Bedeutung eines Wasserstoffatoms hat, deren Salze mit physiologisch verträglichen Basen bedeuten und deren Cyclodextrinclathrate bekannt,
jedoch werden die Ester der vorliegenden Erfindung weder im einzelnen genannt noch besonders hervorgehoben.

Aus EP 30 377 sind 9-Chlor-prostaglandin-Derivate 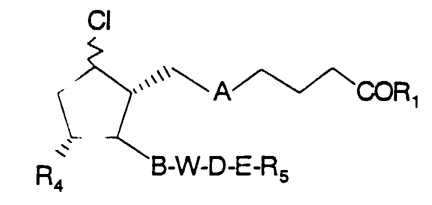 worin
das 9-Chloratom α- oder β-ständig sein kann,
- R₁: den Rest OR₂ mit R₂ in der Bedeutung eines Wasserstoffatoms, Alkyl, Cycloalkyl, Aryl oder eines heterocyclischen Restes oder den Rest NHR₃ mit R₃ in der Bedeutung eines Säurerestes oder Wasserstoffatoms und
- A: eine -CH₂-CH₂- oder cis -CH=CH- Gruppe,
- B: eine -CH₂-CH₂-, trans -CH=CH- oder eine -C≡C- Gruppe,
- W: eine freie oder funktionell abgewandelte Hvdroxymethylengruppe oder eine freie oder funktionell abgewandelte 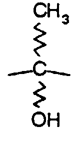 Gruppe, wobei die OH-Gruppe α oder β-ständig sein kann,
- D und E: gemeinsam eine direkte Bindung oder
- D: eine geradkettige oder verzweigtkettige Alkylengruppe mit 1-10 C-Atomen, die gegebenenfalls durch Fluoratome substituiert ist, und
- E: ein Sauerstoff- oder Schwefelatom oder eine direkte Bindung und
- R₄: eine freie oder funktionell abgewandelte Hydroxygruppe,
- R₅: eine Alkyl-, eine Halogen-substituierte Alkyl-, Cycloalkyl-, eine gegebenenfalls substituierte Aryl- oder heterocyclische Gruppe und falls R₁ die Bedeutung einer Hydroxylgruppe hat, deren Salze mit physiologisch verträglichen Basen bedeuten,
bekannt, jedoch werden auch dort die Ester dieser Erfindung weder im einzelnen genannt noch besonders hervorgehoben.

9-Chlor-Prostaglandin-Derivate sind pharmakologisch und medizinisch wertvolle Wirkstoffe, deren Herstellung und Anwendung z.B. zur Zytoprotektion, Ulkusheilung, Hemmung der Magensäuresekretion, Luteolyse, Blutdrucksenkung, oder Plättchenaggregationshemmung in EP 299 914, EP 30 377 und WO 86/05488 beschrieben sind. Diese Substanzen besitzen gegenüber den entsprechenden natürlichen Prostaglandinen bei ähnlichem Wirkungsspektrum eine wesentlich verbesserte Spezifität und längere Wirkung.

Es wurde nun gefunden, daß die neuen 9-Chlor-prostaglandinester und -amide eine hohe Wirkspezifität, bessere Wirksamkeit, längere Wirkdauer und vor allen Dingen höhere Stabilität als die natürlichen Prostaglandine besitzen.

Es wurde ebenfalls gefunden, daß Ester bzw. Amide von 9-Chlor-Δ⁵- bzw. 9-Chlor-3-oxa-Δ⁵-prostaglandinen für die äußeren Anwendungen, insbesondere zur Behandlung erhöhten Augeninnendruckes (Glaukom) besonders geeignet sind. Diese Anwendung wird in dem oben angeführten Stand der Technik nicht erwähnt.

Die Erfindung betrifft pharmazeutische Präparate enthaltend Ester oder Amide von 9-Chlor-prostaglandinen der Formel I 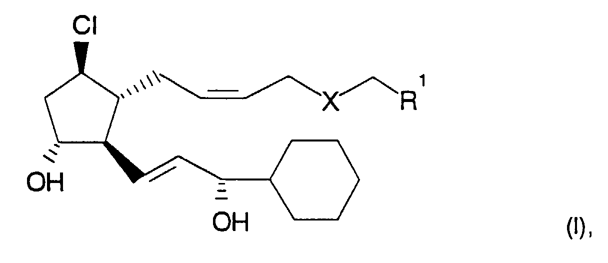 worin
- X: Sauerstoff oder CH₂,
- R¹ COOR²,: wobei R² einen gegebenenfalls substituierten C₁-C₁₀-Alkyl-, C₃-C₁₀-Cycloalkyl-,C₆-C₁₀-Aryl- oder C₆-C₁₀-Ar(C₁-C₄)-alkyl-Rest darstellt, oder CONHR³ mit R³ in der Bedeutung eines gegebenenfalls substituierten C₁-C₁₀-Alkyl-Restes,
bedeuten,
zur Behandlung erhöhten Augeninnendruckes sowie pharmazeutische Präparate enthaltend 9-Chlor-prostaglandinester oder -amide der allgemeinen Formel (I) 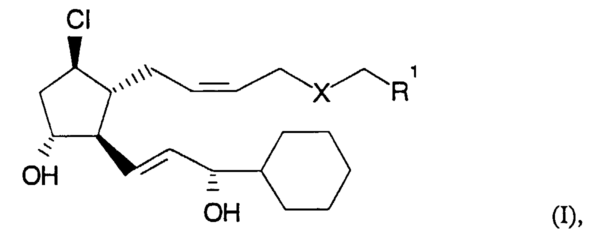 worin
- X: Sauerstoff oder CH₂,
- R¹ COOR²,: wobei R² einen gegebenenfalls substituierten Phenacyl-Rest darstellt, oder CONHR³
mit R³ in der Bedeutung eines gegebenenfalls substituierten C₁-C₁₀-Alkyl-Restes,
bedeuten,
als medizinisch wertvolle Wirkstoffe zur Anwendung für z.B. die Behandlung erhöhten Augeninnendruckes, Zytoprotektion, Ulkusheilung, Hemmung der Magensäuresekretion, Luteolyse, Blutdrucksenkung oder Plättchenaggregationshemmung.

Die Erfindung betrifft außerdem 9-Chlor-prostaglandinester und -amide der allgemeinen Formel I 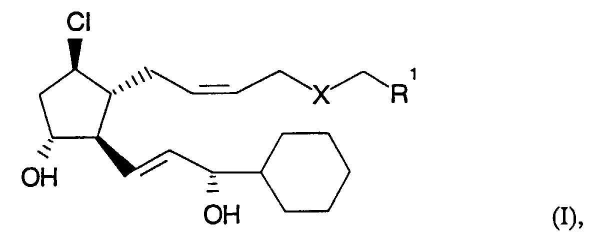 worin
- X: Sauerstoff oder CH₂,
- R¹ COOR²,: wobei R² einen gegebenenfalls substituierten Phenacyl-Rest darstellt, oder CONHR³
mit R³ in der Bedeutung eines gegebenenfalls substituierten C₁-C₁₀-Alkyl-Restes,
bedeuten.

Die Erfindung betrifft auch die Verwendung der in diesen pharmazeutischen Präparaten genannten 9-Chlorprostaglandinderivaten zur Herstellung von Arzneimitteln zur Behandlung des erhöhten Augeninnendrucks.

Es wurde weiterhin gefunden, daß die nicht in EP 299 914 und EP 30 377 genannten, erfindungsgemäßen Ester überraschenderweise besonders für die lokale Anwendung geeignet sind, sowie diuretische Wirkung zeigen und die Nierendurchblutung fördern.

Die Erfindung betrifft somit auch Ester von 9-Chlor-prostaglandinen der allgemeinen Formel I 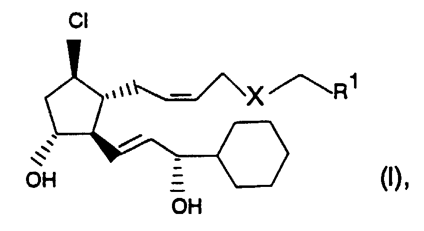 worin
- X: Sauerstoff oder CH₂, und
- R¹: COOR², wobei R² einen gegebenenfalls substituierten C₂-C₃-Alkyl-, Cyclopentyl-, Cyclohexyl-, Phenyl- oder Benzyl-Rest darstellt, bedeuten und deren Cyclodextrinclathrate.

Als Substituenten für den Phenacylrest R² kommen geradkettige oder verzweigte C₁-C₄-Alkyl-Reste wie Methyl, Ethyl, Propyl, Isopropyl, Butyl, Isobutyl und tert.-Butyl sowie die Halogenatome Chlor, Brom und Iod in Betracht.

Als Alkylgruppen R² sind gerade oder verzweigte Alkylgruppen mit 1-10 C-Atomen zu betrachten, wie beispielsweise Methyl, Ethyl, Propyl, Butyl, Isobutyl, tert.-Butyl, Pentyl, Isopentyl, Neopentyl, Hexyl, Heptyl, Decyl. Die Alkylgruppen R² können gegebenenfalls mehrfach substituiert sein durch Halogenatome, Hydroxygruppen, Alkoxygruppen, Dialkylamino- und Trialkylammoniumgruppen, oder gegebenenfalls substituierte Aryl- bzw. Aroylgruppen. Als Substituenten seien beispielsweise genannt Fluor, Chlor oder Brom, Hydroxy, Methoxy, Ethoxy, Dimethylamino, Diethylamino, Trimethylammonium, Phenyl, Benzoyl, Brombenzoyl, Iodbenzoyl, Methylbenzoyl. Als bevorzugte Alkylgruppen R² sind solche mit 1-5 C-Atomen, wie z.B. Methyl, Ethyl, Propyl, Isobutyl, Butyl, tert.-Butyl und als bevorzugte Substituenten Fluor, Hydroxy, Methoxy und Benzoyl zu nennen. Besonders bevorzugte Substituenten sind Hydroxy und Benzoyl.

Als Alkylgruppen R³ sind geradkettige oder verzweigte Alkylgruppen mit 1-10 C-Atomen zu betrachten, wie beispielsweise Methyl, Ethyl, Propyl, Isopropyl, Butyl, Isobutyl, tert.-Butyl, Pentyl, Isopentyl, Neopentyl, Hexyl, Heptyl. Die Alkylgruppen R³ können gegebenenfalls ein- bis mehrfach substituiert sein durch Halogenatome, Hydroxygruppen, Alkoxygruppen, oder gegebenenfalls substituierte Arylgruppen. Als Substituenten seien beispielsweise genannt Fluor, Chlor oder Brom, Hydroxy, Methoxy, Ethoxy, Phenyl. Als bevorzugte Alkylgruppen R³ sind solche mit 1-5 C-Atomen, wie z.B. Methyl, Ethyl, Propyl, Isopropyl, Isobutyl, Butyl, und als bevorzugte Substituenten Fluor, Hydroxy und Methoxy zu nennen.

Als Arylgruppen R² kommen sowohl substituierte wie auch unsubstituierte Arylgruppen in Betracht, wie beispielsweise Phenyl, 1-Naphtyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Als Aralkylgruppe R² wird die Benzylgruppe bevorzugt.

Die Cycloalkylgruppe R² kann im Ring 3-10, vorzugsweise 5 und 6 Kohlenstoffatome enthalten. Die Ringe können durch Alkylgruppen mit 1-4 Kohlenstoffatomen substituiert sein. Beispielsweise seien genannt Cyclopentyl, Cyclohexyl, Methylcyclohexyl und Adamantyl.

Für die Arylgruppen als Substituenten der Alkylreste R³ kommen sowohl substituierte wie auch unsubstituierte Arylgruppen in Betracht, wie beispielsweise Phenyl, 1-Naphtyl und 2-Naphthyl, die jeweils substituiert sein können durch 1-3 Halogenatome, eine Phenylgruppe, 1-3 Alkylgruppen mit jeweils 14 C-Atomen, eine Chlormethyl, Fluormethyl-, Trifluormethyl-, Carboxyl-, Hydroxy- oder Alkoxygruppe mit 14 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy.

Die Erfindung betrifft ferner ein Verfahren zur Herstellung der 9-Chlor-prostaglandinester und -amide der Formel I, dadurch gekennzeichnet, daß man
a) eine Verbindung der Formel II 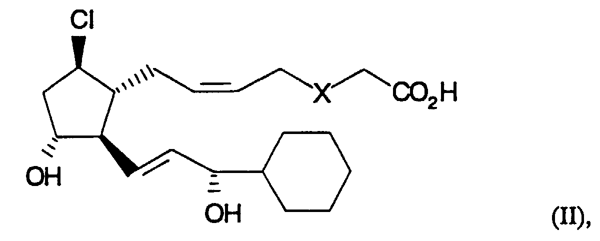 worin
   - X: die oben angegebene Bedeutung aufweist,
   unter Zusatz einer geeigneten Base mit einem gegebenenfalls substituierten Halogenacetophenonderivat der allgemeinen Formel III 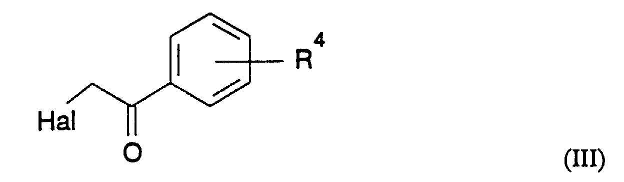 worin
   - R⁴: C₁-C₄-Alkyl-, Chlor, Brom oder Iod und
   - Hal: Chlor oder Brom bedeuten,
   in einem polaren Lösungsmittel zu Verbindungen der allgemeinen Formel (Ia) umsetzt, 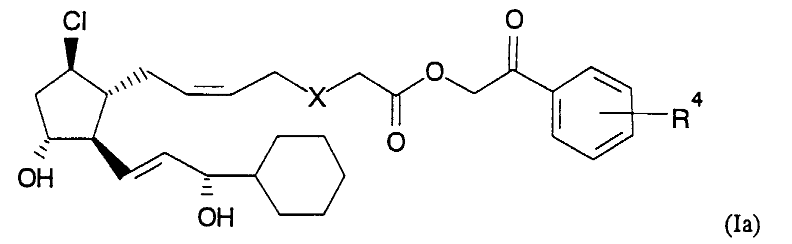 oder
b) einen Alkylester der allgemeinen Formel Ib 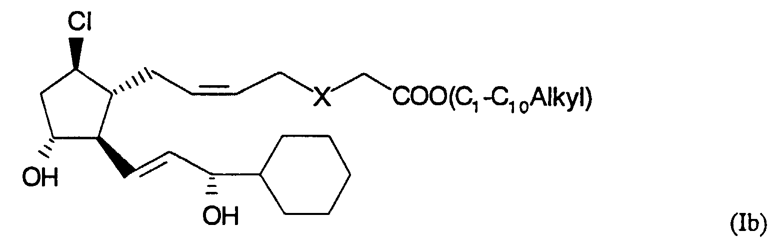 worin
   - X: die oben angegebene Bedeutung aufweist,
   ohne Lösungsmittel mit einem Amin der allgemeinen Formel IV

   H₂N―R³ (IV)

   zu Verbindungen der allgemeinen Formel Ic 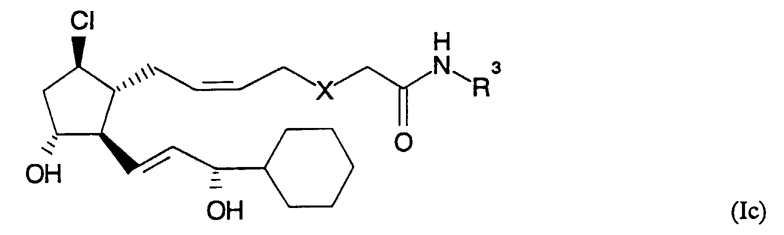 umsetzt.

Als Base für die Umsetzung von Verbindungen der allgemeinen Formel II mit Verbindungen der allgemeinen Formel III sind die dem Fachmann bekannten Basen, z.B. Triethylamin, Diisopropylethylamin, Diazabicycloundecen, Diazabicyclononan, N,N-Dimethylaminopyridin, Kaliumcarbonat oder Cäsiumcarbonat besonders geeignet.

Die Umsetzung kann in allen polaren Lösungsmitteln, z.B. Aceton, Acetonitril, Dimethylformamid oder Dimethylsulfoxid erfolgen.

Die Einführung der Estergruppe CO₂R² für R¹, bei welcher R² eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterungen mit Diazokohlenwasserstoffen erfolgt z. B. dadurch, daß man eine Lösung des Diazokohlenwasserstoffes in einem inerten Lösungsmittel, vorzugsweise in Diethylether, mit der Carboxyverbindung in dem gleichen oder in einem anderen Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seite 389-394(1954)].

Die Einführung der Estergruppe CO₂R² für R¹, bei welcher R² eine Alkylgruppe mit 1-10 C-Atomen darstellt, die gegebenenfalls auch substituiert sein kann, erfolgt nach den dem Fachmann bekannten Methoden. Die Carboxyverbindungen werden beispielsweise mit dem entsprechenden Alkylhalogenid, bevorzugt Alkylbromid oder Alkyliodid, in Gegenwart einer Base, wie beispielsweise Triethylamin, Diazabicylononan (DBN), Diazabicycloundecan (DBU) in einem inerten Lösungsmittel, wie beispielsweise Acetonitril, Tetrahydrofuran, Methylenchlorid oder Dimethylformamid bei Temperaturen zwischen -80°C und 100°C, vorzugsweise bei 0°C bis 30°C, verestert.

Die Synthese der Phenacylester (5Z,13E)-(9R,11R,15S)-9-Chlor-15-cyclohexyl-11,15-dihydroxy-16,17,18,19,20-pentanor-5,13-prostadiensäurephenacylester und (5Z,13E)-(9R,11R,15S)-9-Chlor-3-oxa-15-cyclohexyl-11,15-dihydroxy-16,17,18,19,20-pentanor-5,13-prostadiensäurephenacylester in den Beispielen 1 und 3 ist allgemein anwendbar als Verfahren zur Herstellung von Phenacylestern von 9-Chlor-Prostaglandinderivaten.

Speziell Ester tertiärer Alkohole werden aus der Carboxyverbindung in einem inerten Lösungsmittel vorzugsweise Methylenchlorid unter Säurekatalyse und Bortrifluorid-Etherat mit dem entsprechenden Alken bei Temperaturen zwischen -100°C und +50°C, vorzugsweise zwischen -78°C und 0°C, hergestellt. Für die Säurekatalyse wird vorzugsweise Orthophosphorsäure verwendet.

Die Einführung der Estergruppe CO₂R² für R¹, bei welcher R² eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, Diazabicyclononan (DBN), Diazabicycloundecan (DBU), in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30°C und +50°C, vorzugsweise bei Temperaturen zwischen 0°C und Raumtemperatur, durchgeführt.

Die Einführung der Amidgruppe CONHR³ für R¹ erfolgt vorzugsweise aus der Estergruppe durch Aminolyse mit dem entsprechenden Amin R³NH₂, d.h. der entsprechende Ester wird vorzugsweise ohne Lösungsmittel oder in einem inerten Lösungsmittel, wie beispielsweise Acetonitril oder Dimethylformamid, mit dem entsprechenden Amin für mehrere Stunden zwischen 40°C und 140°C, vorzugsweise 60°C bis 90°C erhitzt.

Die Synthese der Amide (5Z,13E)-(9R,11R,15S)-9-Chlor-15-cyclohexyl-11,15-dihydroxy-16,17,18,19,20-pentanor-5,13-prostadiensäure-(3-hydroxypropyl)-amid und (5Z,13E)-(9R,11R,15S)-9-Chlor-3-oxa-15-cyclohexyl-11,15-dihydroxy-16,17,18,19,20-pentanor-5,13-prostadiensäure-(3-hydroxypropyl)-amid in den Beispielen 2 und 4 ist allgemein anwendbar als Verfahren zur Herstellung der Amide von 9-Chlor-Prostaglandinderivaten.

Die für die Synthese der Ester und Amide benötigten Ausgangsmaterialien sind nach Vorschriften aus EP 299 914 und WO 86/05488 zu erhalten.

Cyclodextrinclathrate können analog einer Vorschrift in WO 87/05294 erhalten werden.

Die Erfindung betrifft außerdem ein Verfahren zur Herstellung von Estern der 9-Chlorprostaglandine der Formel I worin
- X: Sauerstoff oder CH₂, und
- R¹: COOR², wobei R² einen gegebenenfalls substituierten C₂-C₃-Alkyl-, Cyclopentyl, Cyclohexyl-, Phenyl- oder Benzyl-Rest darstellt, bedeuten,
dadurch gekennzeichnet, daß man in an sich bekannter Weise eine Verbindung der Formel II 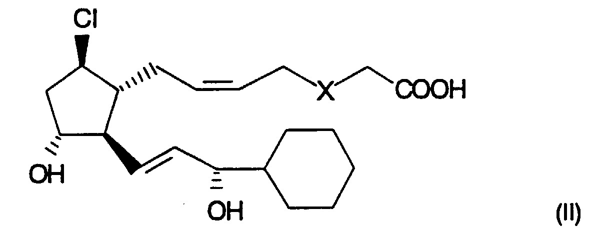 worin X die oben angegebene Bedeutung hat,

**A.** entweder in Gegenwart einer geeigneten Base in einem polaren Lösungsmittel mit einer Verbindung der allgemeinen Formel III'

Hal-R² (III')

worin R² die oben angegebenen Bedeutung hat, jedoch nicht Phenyl bedeutet, und Hal für Chlor, Brom oder Iod steht,bei Temperaturen zwischen -80°C und 100°C umsetzt, und den entstandenen Ester isoliert oder
**B.**in Gegenwart einer geeigneten Base in einem polaren Lösungsmittel nach Aktivierung mit einem Carbodiimid wie z. B. Dicyclohexylcarbodiimid mit Phenol bei Temperaturen zwischen -30°C und 50°C umsetzt und den entstandenen Ester isoliert.

Als Base für die Umsetzung von Verbindungen der allgemeinen Formel II mit Verbindungen der allgemeinen Formel III' oder mit Phenol sind die dem Fachmann bekannten Basen, z.B. Diazabicyclononan, Diazabicycloundecen, Diisopropylethylamin, N,N-Dimethylaminopyridin, Pyridin, Triethylamin, Kaliumcarbonat oder Cäsiumcarbonat besonders geeignet.

Die Umsetzungen können in polaren Lösungsmitteln, z.B. Aceton, Acetonitril, Chloroform, Diethylether, Dimethylformamid, Dimethylsulfoxid, Methylenchlorid oder Tetrahydrofuran erfolgen.

Die entsprechend dieser Erfindung hergestellten Ester und Amide von 9-Chlor-Prostaglandinanaloga sind chemisch stabile PGD-Derivate. Sie stellen wertvolle Pharmaka dar, da sie bei ähnlichem Wirkungsspektrum eine wesentlich verbesserte (höhere Spezifität) und vor allem wesentlich längere Wirkung aufweisen als die entsprechenden natürlichen Prostaglandine.

Sie sind als medizinisch wertvolle Wirkstoffe zur Anwendung für z.B. Blutdrucksenkung, die Förderung der Hautdurchblutung, Luteolyse, Hemmung der Magensäuresekretion, Plättchenaggregationshemmung, Ulkusheilung oder Zytoprotektion geeignet.

Die erfindungsgemäßen Verbindungen können auch in Kombination, z.B. mit ß-Blockern, Diuretika, Phosphodiesterasehemmern, Calciumantagonisten, Thromboxanantagonisten, Thromboxansynthetase- und Cyclooxygenasehemmern, gerinnungshemmenden Substanzen, wie auch Fibrinolytika, Leukotrienantagonisten, Leukotriensynthetasehemmern und Antigestagenen, verwendet werden.

Besonders geeignet sind die erfindungsgemäßen Verbindungen zur lokalen Anwendung wie z.B. zur Förderung der Hautdurchblutung und zur Senkung erhöhten Augeninnendruckes (Glaukom), sowie zur Förderung der Nierendurchblutung und zur Verwendung als Diuretikum.

Bei Kaninchen bewirkt die lokale Applikation der Verbindungen eine Senkung des Augeninnendruckes.

Bei Affen mit experimentellem Glaukom bewirkt die lokale Applikation der Verbindungen eine Normalisierung des pathologisch erhöhten Augeninnendruckes.

Die Einzeldosis der Verbindungen für die Anwendung zur Behandlung erhöhten Augeninnendruckes ist 1 ng - 100 µg / Auge, einmal oder mehrmals täglich, wenn sie am menschlichen Patienten lokal verabreicht werden.

Für die lokale Applikation , wie z. B. für die Anwendung zur Behandlung erhöhten Augeninnendruckes sind beispielsweise Lösungen, Lotionen oder Salben geeignet.

Die Dosis der Verbindungen bei lokaler Anwendung zur Förderung der Hautdurchblutung ist 5-500 ng/cm², wenn sie am menschlichen Patienten verabreicht werden.

Für die lokale Applikation für die Anwendung zur Förderung der Hautdurchblutung sind beispielsweise Lösungen, Lotionen, Salben, Cremes oder Pflaster geeignet.

Die Erfindung betrifft auch Arzneimittel auf Basis der Verbindungen der Formel I, sowie deren Cyclodextrinclathrate, mit den üblichen Hilfs- und Trägerstoffen.

Die erfindungsgemäßen Wirkstoffe sollen in Verbindung mit den in der Galenik bekannten und üblichen Hilfsstoffen, z.B. zur Herstellung von Präparaten zur Förderung der Hautdurchblutung, zur Behandlung des erhöhten Augeninnendruckes (Glaukom), zur Förderung der Nierendurchblutung oder zur Verwendung als Diuretikum dienen.

Die folgenden Beispiele sollen die Erfindung näher erläutern, ohne daß damit eine Begrenzung vorgenommen werden soll.

### Beispiel 1

### (5Z,13E)-(9R,11R,15S)-9-Chlor-15-cyclohexyl-11,15-dihydroxy-16,17,18,19,20-pentanor-5,13-prostadiensäurephenacylester

Zu einer Lösung aus 100 mg (5Z,13E)-(9R,11R,15S)-9-Chlor-15-cyclohexyl-11,15-dihydroxy-16,17,18,19,20-pentanor-5,13-prostadiensäure und 75 mg w-Bromacetophenon in 2.6 ml Acetonitril gibt man 0.057 ml Triethylamin in 1.25 ml Aceton und rührt anschließend 18 Stunden bei 24°C unter Argon. Man gibt dann nochmal 40 mg w-Bromacetophenon zu und rührt nochmals 16 Stunden bei 24°C unter Argon. Dann verdünnt man mit 80 ml Essigester, wäscht einmal mit 10 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 114 mg der Titelverbindung als farbloses Öl.

IR (CHCl₃): 3607, 3400 (br.), 3065, 3030, 3003, 2928, 2857, 1742, 1705, 1600, 1450, 1376, 1155, 1084, 1002, 972 cm⁻¹.

### Beispiel 2

### (5Z,13E)-(9R,11R,15S)-9-Chlor-15-cyclohexyl-11,15-dihydroxy-16,17,18,19,20-pentanor-5,13-prostadiensäure-(3-hydroxypropyl)-amid

86.4 mg (5Z,13E)-(9R,11R,15S)-9-Chlor-15-cyclohexyl-11,15-dihydroxy-16,17,18,19,20-pentanor-5,13-prostadiensäuremethylester aus Beispiel 1 werden mit 157.3 mg 3-Amino-1-propanol versetzt und 24 Stunden bei 80°C unter Argon gerührt. Das Reaktionsgemisch reinigt man durch Säulenchromatographie an Kieselgel. Mit CH₂Cl₂ / 0 - 50% Methanol als Elutionsmittel erhält man 30.5 mg der Titelverbindung als farbloses Öl.

IR (Flüssig-Kap.): 3310 (br.), 3100, 3008, 2927, 2853, 1643, 1630, 1553, 1448, 1348, 1260, 1080, 1072, 1003, 970 cm⁻¹.

### Beispiel 3

### (5Z,13E)-(9R,11R,15S)-9-Chlor-3-oxa-15-cyclohexyl-11,15-dihydroxy-16,17,18,19,20-pentanor-5,13-prostadiensäurephenacylester

Zu einer Lösung aus 92 mg (5Z,13E)-(9R,11R,15S)-9-Chlor-3-oxa-15-cyclohexyl-11,15-dihydroxy-16,17,18,19,20-pentanor-5,13-prostadiensäure und 105 mg w-Bromacetophenon in 2.5 ml Acetonitril gibt man 0.052 ml Triethylamin in 1.0 ml Aceton und rührt anschließend 18 Stunden bei 24°C unter Argon. Dann verdünnt man mit 60 ml Essigester, wäscht zweimal mit je 10 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 82.6 mg der Titelverbindung als farbloses Öl.

IR (CHCl₃): 3605, 3410 (br.), 3030, 2998, 2927, 2853, 1763, 1730, 1704, 1600, 1450, 1375, 1245, 1190, 1180, 1127, 1000, 972 cm⁻¹.

### Beispiel 4

### (5Z,13E)-(9R,11R,15S)-9-Chlor-3-oxa-15-cyclohexyl-11,15-dihydroxy-16,17,18,19,20-pentanor-5,13-prostadiensäure-(3-hydroxypropyl)-amid

80 mg (5Z,13E)-(9R,11R,15S)-9-Chlor-3-oxa-15-cyclohexyl-11,15-dihydroxy-16,17,18,19,20-pentanor-5,13-prostadiensäuremethylester aus Beispiel 6 werden mit 149.7 mg 3-Amino-1-propanol versetzt und 24 Stunden bei 80°C unter Argon gerührt. Das Reaktionsgemisch reinigt man durch Säulenchromatographie an Kieselgel. Mit CH₂Cl₂ / 0 - 50% Methanol als Elutionsmittel erhält man 49.6 mg der Titelverbindung als farbloses Öl.

IR (Flüssig-Kap.): 3362 (br.), 3023, 2928, 2854, 1657, 1543, 1450, 1335, 1304, 1273, 1102, 1006, 972 cm⁻¹.

### Beispiel 5

### (5Z,13E)-(9R,11R,15S)-9-Chlor-15-cyclohexyl-11,15-dihydroxy-16,17,18,19,20-pentanor-5,13-prostadiensäuremethylester

Zu einer Lösung von 27 mg (5Z,13E)-(9R,11R,15S)-9-Chlor-15-cyclohexyl-11,15-dihydroxy-16,17,18,19,20-pentanor-5,13-prostadiensäure in 10 ml Methylenchlorid tropft man bei 0°C unter Argon eine etherische Diazomethan-Lösung bis zur bleibenden Gelbfärbung, rührt 5 Minuten bei 0°C und engt anschließend im Vakuum ein. Den so erhaltenen Rückstand reinigt man durch Chromatographie an Kieselgel. Mit Hexan / 0-80% Essigester erhält man 24 mg der Titelverbindung als farbloses Öl.

IR (CHCl₃): 3610, 3420 (br.), 3030, 3002, 2931, 2857, 1732, 1447, 1435, 1365, 1314, 1230 (br.), 1083, 995, 970 cm⁻¹.

### Beispiel 6

### (5Z,13E)-(9R,11R,15S)-9-Chlor-15-cyclohexyl-11,15-dihydroxy-16,17,18,19,20-pentanor-5,13-prostadiensäureethylester

Zu 250 mg (5Z,13E)-(9R,11R,15S)-9-Chlor-15-cyclohexyl-11,15-dihydroxy-16,17,18,19,20-pentanor-5,13-prostadiensäure gibt man 6.25 ml einer Lösung aus Acetonitril, Diazabicycloundecan (DBU) und Iodethan (50 ml Acetonitril, 0.8 ml DBU, 0.8 ml Iodethan) und rührt 22 Stunden bei 24°C unter Argon. Anschließend verdünnt man mit 150 ml Essigester, wäscht einmal mit 5%iger Natriumhydrogencarbonat-Lösung, dreimal mit je 10 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 129 mg der Titelverbindung als farbloses Öl.

IR (CHCl₃): 3608, 3410 (br.), 3020, 3000, 2980, 2928, 2857, 1726, 1450, 1376, 1245, 1095, 1085, 973 cm⁻¹.

### Beispiel 7

### (5Z,13E)-(9R,11R,15S)-9-Chlor-15-cydohexyl-11,15-dihydroxy-16,17,18,19,20-pentanor-5,13-prostadiensäureisopropylester

Zu 100 mg (5Z,13E)-(9R,11R,15S)-9-Chlor-15-cyclohexyl-11,15-dihydroxy-16,17,18,19,20-pentanor-5,13-prostadiensäure gibt man 2.5 ml einer Lösung aus Acetonitril, Diazabicycloundecan (DBU) und 2-Iodpropan (50 ml Acetonitril, 0.8 ml DBU, 0.8 ml 2-Iodpropan) und rührt 22 Stunden bei 24°C unter Argon. Anschließend verdünnt man mit 70 ml Essigester, wäscht zweimal mit je 10 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 50.1 mg der Titelverbindung als farbloses Öl.

IR (CHCl₃): 3609, 3415 (br.), 3030, 3000, 2983, 2928, 2857, 1723, 1453, 1377, 1247, 1207, 1085, 973 cm⁻¹.

### Beispiel 8

### (5Z,13E)-(9R,11R,15S)-9-Chlor-3-oxa-15-cyclohexyl-11,15-dihydroxy-16,17,18,19,20-pentanor-5,13-prostadiensäuremethylester

Zu einer Lösung von 300 mg (5Z,13E)-(9R,11R,15S)-9-Chlor-3-oxa-15-cyclohexyl-11,15-dihydroxy-16,17,18,19,20-pentanor-5,13-prostadiensäure in 30 ml Methylenchlorid tropft man bei 0°C unter Argon eine etherische Diazomethan-Lösung bis zur bleibenden Gelbfärbung, rührt 10 Minuten bei 0°C und engt anschließend im Vakuum ein. Den so erhaltenen Rückstand reinigt man durch Chromatographie an Kieselgel. Mit Hexan / 0-80% Essigester erhält man 235 mg der Titelverbindung als farbloses Öl.

IR (CHCl₃): 3607, 3408 (br.), 3033, 3000, 2927, 2854, 1752, 1450, 1442, 1377, 1347, 1280, 1123, 997, 973 cm⁻¹.

### Beispiel 9

### (5Z,13E)-(9R,11R,15S)-9-Chlor-3-oxa-15-cyclohexyl-11,15-dihydroxy-16,17,18,19,20-pentanor-5,13-prostadiensäureethylester

Zu 92 mg (5Z,13E)-(9R,11R,15S)-9-Chlor-3-oxa-15-cyclohexyl-11,15-dihydroxy-16,17,18,19,20-pentanor-5,13-prostadiensäure gibt man 2.3 ml einer Lösung aus Acetonitril, Diazabicycloundecan (DBU) und Iodethan (50 ml Acetonitril, 0.8 ml DBU, 0.8 ml Iodethan) und rührt 18 Stunden bei 24°C unter Argon. Dann nochmals mit 0.73 ml einer Lösung aus 100 mg DBU in 10 ml Acetonitril versetzt und weitere 20 Stunden bei 24°C unter Argon gerührt. Anschließend verdünnt man mit 70 ml Essigester, wäscht einmal mit 10 ml einer 5%iger Natriumhydrogencarbonat-Lösung, dreimal mit je 10 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 75.5 mg der Titelverbindung als farbloses Öl.

IR (CHCl₃): 3605, 3415 (br.), 3030, 2997, 2985, 2926, 2853, 1745, 1447, 1380, 1300, 1273, 1125, 1020, 997, 972 cm⁻¹.

### Beispiel 10

### (5Z,13E)-(9R,11R,15S)-9-Chlor-3-oxa-15-cyclohexyl-11,15-dihydroxy-16,17,18,19,20-pentanor-5,13-prostadiensäureisopropylester

Zu 190 mg (5Z,13E)-(9R,11R,15S)-9-Chlor-3-oxa-15-cyclohexyl-11,15-dihydroxy-16,17,18,19,20-pentanor-5,13-prostadiensäure gibt man 4.7 ml einer Lösung aus Acetonitril, Diazabicycloundecan (DBU) und 2-Iodpropan (50 ml Acetonitril, 0.8 ml DBU, 0.8 ml 2-Iodpropan) und rührt 18 Stunden bei 24°C unter Argon. Anschließend verdünnt man mit 70 ml Essigester, wäscht zweimal mit je 20 ml einer Mischung aus gesättigter Natriumchlorid-Lösung und Wasser (1:1), trocknet über Natriumsulfat und engt im Vakuum ein. Das Rohprodukt reinigt man durch Säulenchromatographie an Kieselgel. Mit Hexan / 0 - 80% Essigester als Elutionsmittel erhält man 83.1 mg der Titelverbindung als farbloses Öl.

IR (CHCl₃): 3605, 3410 (br.), 3030, 3000, 2983, 2925, 2853, 1738, 1448, 1376, 1125, 1102, 997, 973 cm⁻¹.

### Beispiel 11

### (5Z,13E)-(9R,11R,15S)-9-Chlor-15 cyclohexyl-11,15-dihydroxy-3-oxa-16,17,18,19,20-pentanor-5,13-prostadiensäure-tert.-butylester

Zu einer Lösung von 2,54g (5Z,13E)-(95,11R,15S)-15-Cyclohexyl-9-hydroxy-3-oxa-11,15-bis(tetrahydropyran-2-yloxy)-16,17,18,19,20-pentanor-5,13-prostadiensäure-tert.butylester in 25ml Pyridin gibt man bei 0°C 773mg Methansulfonsäurechlorid. Man rührt 4 Stunden bei 20°C und gibt dann die Lösung zu einer Suspension von 25,37g Tetrabutylammoniumchlorid in 20ml Toluol. Nach 15 stündigem Rühren bei 0°C wird noch 7 Stunden bei 40°C gerührt. Anschließend gibt man auf 250ml Eiswasser und extrahiert dreimal mit je 100ml Ether. Nachdem die organische Phase je zweimal mit 40ml gesättigter Natriumchloridlösung gewaschen, über Magnesiumsulfat getrocknet und im Vakuum eingedampft wird, erhält man einen Rückstand, den man an Kieselgel mit Hexan/0-40% Ether chromatographiert. Man erhält 2,32g öligen (5Z, 13E)-(9R, 11R, 15S)-9-Chlor-15cyclohexyl-3-oxa-11,15-bis(tetrahydropyran-2-yloxy)-16,17,18,19,20-pentanor-5,13-prostadiensäure-tert.-butylester. Zur Abspaltung der Schutzgruppen rührt man den erhaltenen Ester mit 85ml einer Mischung aus Essigsäure/ Wasser/Tetrahydrofuran (65/35/10) 24 Stunden bei 20°C. Nach Zugabe von Toluol und Eindampfen der Lösung im Vakuum chromatographiert man den Rückstand an Kieselgel. Mit Methylenchlorid/0,5% Aceton als Elutionsmittel erhält man 915mg der Titelverbindung als farbloses Öl.IR (CHCl₃): 3605, 3410, 2928, 1742, 1020, 974 cm⁻¹.

### Beispiel 12

### Messung der Hautdurchblutung

Hairless Ratten (weiblich) werden gewogen (200-250g), mit 0,5ml 25% Urethan in 0,9% NaCl /100g/kg (2/3 i.p., 1/3 s.c.) narkotisiert und auf eine vorgeheizte Wärmeplatte der Fa. Jowitherm gelegt.

Auf dem Abdomen wird eine kreisförmige Fläche von 34mm Durchmesser ca. 2cm unterhalb der Sternumspitze markiert. Pro Tier wird eine Testsubstanz appliziert, die mit dem Randomisierungsprogramm RDRK zugeordnet wurde. Als Lösungsmittel wird 5% Isopropylmyristat in Ethanol verwendet. 20 ml der jeweiligen Testlösung werden mit einer Eppendorf-Pipette gleichmäßig aufgetragen. Danach wird die behandelte Haut nochmals in drei Meßflächen von jeweils 18mm Druchmesser unterteilt und markiert. Die Hautdurchblutung wird 2 und 4 Stunden ( und gegebenenfalls 6 Stunden) nach der Applikation mit einem Laser Doppler Flowmeter (Firma Perimed) gemessen. Es werden an jedem Tier Dreifachmessungen an den markierten Stellen durchgeführt.

| **9-Chlorprostaglandin aus dem Beispiel** | **Applizierte Konzentration in Prozent** | **Steigerung der Hautdurchblutung in Prozent** |
|---|---|---|
| Beispiel 1 | 0,001 | 50 |
| | 0,003 | 160 |
| Beispiel 3 | 0,001 | 139 |
| | 0,003 | 146 |

### Beispiel 13

### Messung der Hautdurchblutung

Die Hautdurchblutung wurde mit der nichtinvasiven Laser-Doppler Methode bestimmt. Die Messsungen wurden 4 Stunden nach der lokalen Applikation der Substanzen auf der Bauchhaut von narkotisierten ( Urethan ) haarlosen Ratten* durchgeführt. Als Lösungsmittel wurde Isopropylmyristat in Ethanol verwendet ( 5/95; v/v ).

| **9-Chlorprostaglandin aus dem Beispiel** | **Applizierte Konzentration in Prozent** | **Steigerung der Hautdurchblutung in Prozent** |
|---|---|---|
| Beispiel 6 | 0,001 | 115 |
| | 0,003 | 197 |
| Beispiel 7 | 0,001 | 93 |
| | 0,003 | 183 |
| Beispiel 9 | 0,001 | 200 |
| | 0,003 | 325 |
| Beispiel 10 | 0,001 | 232 |
| | 0,003 | 307 |

* Für diese Untersuchungen wurden weibliche Ratten (Wister; hairless ) mit einem Körpergewicht von 200-250g verwendet.

## Patentansprüche

1. Pharmazeutische Präparate enthaltend Ester oder Amide von 9-Chlor-prostaglandinen der Formel (I) 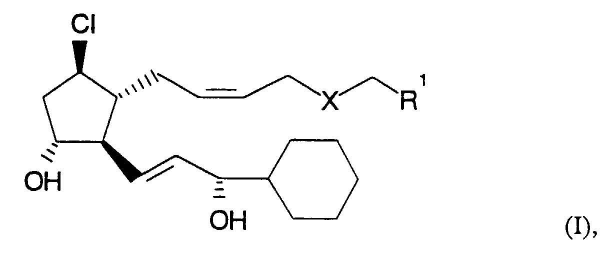 worin
X Sauerstoff oder CH₂,
R¹ COOR², wobei R² einen gegebenenfalls substituierten C₁-C₁₀-Alkyl-, C₃-C₁₀-Cycloalkyl-,C₆-C₁₀-Aryl- oder C₆-C₁₀-Ar(C₁-C₄)-alkyl-Rest darstellt, oder CONHR³ mit R³ in der Bedeutung eines gegebenenfalls substituierten C₁-C₁₀-Alkyl-Restes,
bedeuten,
zur Behandlung erhöhten Augeninnendruckes.

2. Pharmazeutische Präparate enthaltend 9-Chlor-prostaglandinester oder -amide der allgemeinen Formel I 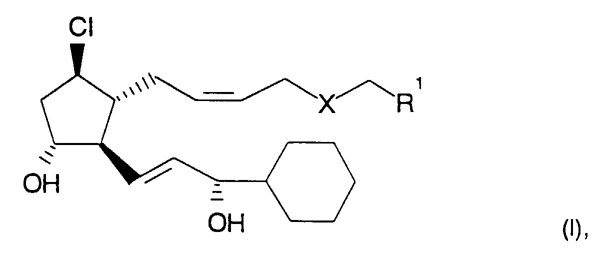 worin
X Sauerstoff oder CH₂,
R¹ COOR², wobei R² einen gegebenenfalls substituierten Phenacyl-Rest darstellt, oder CONHR³
mit R³ in der Bedeutung eines gegebenenfalls substituierten C₁-C₁₀-Alkyl-Restes,
bedeuten,
zur Behandlung erhöhten Augeninnendruckes und zur Zytoprotektion, Ulkusheilung, Hemmung der Magensäuresekretion, Luteolyse, Blutdrucksenkung oder Plättchenaggregationshemmung.

3. Verwendung von 9-Chlorprostaglandinderivaten nach Anspruch 1 und 2 zur Herstellung von Arzneimitteln zur Behandlung des erhöhten Augeninnendruckes.

4. 9-Chlor-prostaglandinester und -amide der allgemeinen Formel (I) 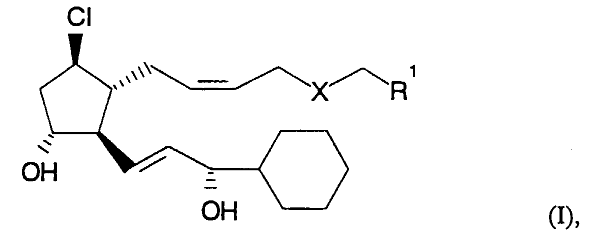 worin
X Sauerstoff oder CH₂,
R¹ COOR², wobei R² einen gegebenenfalls substituierten Phenacyl-Rest darstellt, oder CONHR³
mit R³ in der Bedeutung eines gegebenenfalls substituierten C₁-C₁₀-Alkyl-Restes,
bedeuten,

5. Ester von 9-Chlor-prostaglandinen der allgemeinen Formel I 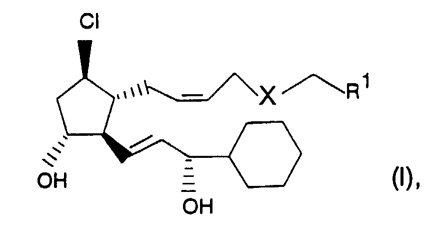 worin
X Sauerstoff oder CH₂, und
R¹ COOR², wobei R² einen gegebenenfalls substituierten C₂-C₃-Alkyl-, Cyclopentyl-, Cyclohexyl-, Phenyl- oder Benzyl-Rest darstellt, bedeuten und deren Cyclodextrinclathrate.

6. Verfahren zur Herstellung der 9-Chlor-prostaglandinester und -amide der Formel I, dadurch gekennzeichnet, daß man
eine Verbindung der Formel II 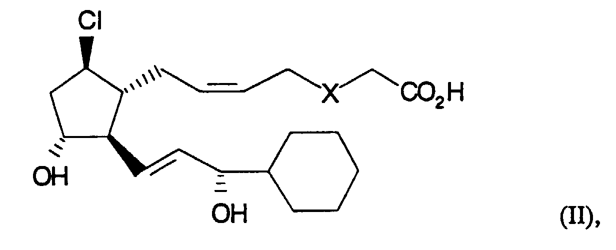 worin
X die oben angegebene Bedeutung aufweist,
unter Zusatz einer geeigneten Base mit einem gegebenenfalls substituierten Halogenacetophenonderivat der allgemeinen Formel III 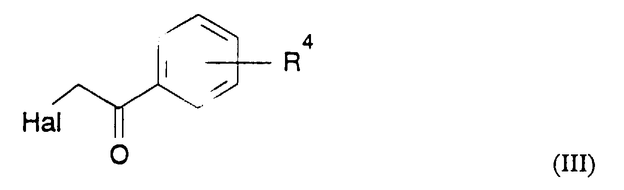 worin
R⁴ C₁-C₄-Alkyl-, Chlor, Brom, oder Iod und
Hal Chlor oder Brom bedeuten,
in einem polaren Lösungsmittel zu Verbindungen der allgemeinen Formel (Ia) umsetzt, 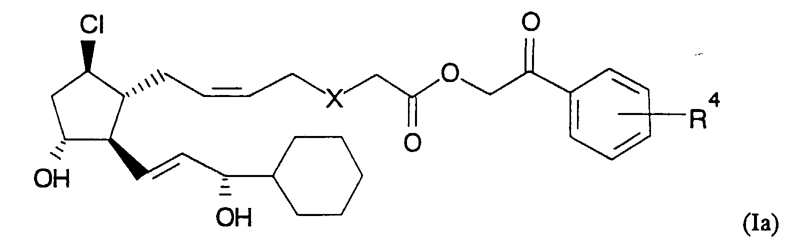 oder
einen Alkylester der allgemeinen Formel Ib 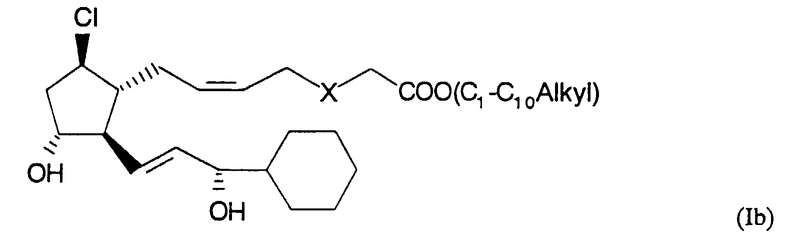 worin
X die oben angegebene Bedeutung aufweist,
ohne Lösungsmittel mit einem Amin der allgemeinen Formel IV
H₂N―R³ (IV)
zu Verbindungen der allgemeinen Formel Ic 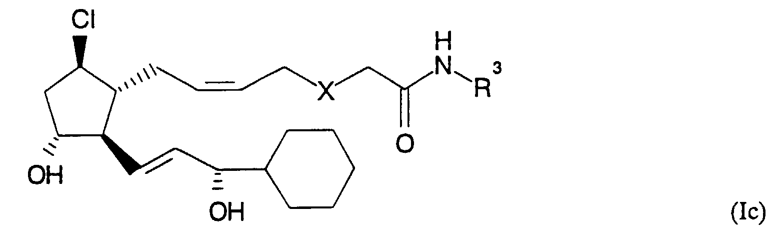 umsetzt.

7. Verfahren zur Herstellung von Estern der 9-Chlor-prostaglandine der Formel I worin
X Sauerstoff oder CH₂, und
R¹ COOR², wobei R² einen gegebenenfalls substituierten C₂-C₃-Alkyl-, Cyclopentyl, Cyclohexyl-, Phenyl- oder Benzyl-Rest darstellt, bedeuten,
dadurch gekennzeichnet, daß man in an sich bekannter Weise eine Verbindung der Formel II 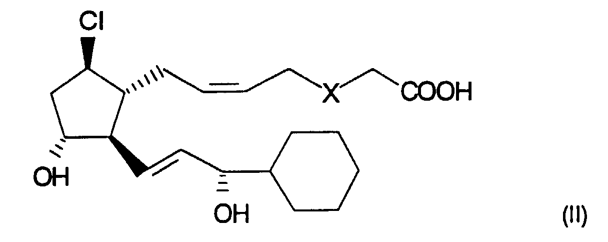 worin X die oben angegebene Bedeutung hat,
**A.** entweder in Gegenwart einer geeigneten Base in einem polaren Lösungsmittel mit einer Verbindung der allgemeinen Formel III'
Hal-R² (III')
worin R² die oben angegebenen Bedeutung hat, jedoch nicht Phenyl bedeutet, und Hal für Chlor, Brom oder Iod steht,bei Temperaturen zwischen -80°C und 100°C umsetzt, und den entstandenen Ester isoliert oder in Gegenwart einer. geeigneten Base in einem polaren Lösungsmittel nach Aktivierung mit einem Carbodiimid wie z. B. Dicyclohexylcarbodiimid mit Phenol bei Temperaturen zwischen -30°C und 50°C umsetzt und den entstandenen Ester isoliert.

8. (5Z,13E)-(9R,11R,15S)-9-Chlor-15-cyclohexyl-11,15-dihydroxy-16,17,18,19,20-pentanor-5,13-prostadiensäurephenacylester

9. (5Z,13E)-(9R,11R,15S)-9-Chlor-15-cyclohexyl-11,15-dihydroxy-16,17,18,19,20-pentanor-5,13-prostadiensäure-(3-hydroxypropyl)-amid

10. (5Z,13E)-(9R,11R,15S)-9-Chlor-3-oxa- 15-cyclohexyl-11,15-dihydroxy-16,17,18,19,20-pentanor-5,13-prostadiensäurephenacylester

11. (5Z,13E)-(9R,11R,15S)-9-Chlor-3-oxa-15-cyclohexyl-11,15-dihydroxy-16,17,18,19,20-pentanor-5,13-prostadiensäure-(3-hydroxypropyl)-amid

12. (5Z,13E)-(9R,11R,15S)-9-Chlor-15-cyclohexyl-11,15-dihydroxy-16,17,18,19,20-pentanor-5,13-prostadiensäuremethylester

13. (5Z,13E)-(9R,11R,15S)-9-Chlor-15-cyclohexyl-11,15-dihydroxy-16,17,18,19,20-pentanor-5,13-prostadiensäureethylester

14. (5Z,13E)-(9R,11R,15S)-9-Chlor-15-cyclohexyl-11,15-dihydroxy-16,17,18,19,20-pentanor-5,13-prostadiensäureisopropylester

15. (5Z,13E)-(9R,11R,15S)-9-Chlor-3-oxa-15-cyclohexyl-11,15-dihydroxy-16,17,18,19,20-pentanor-5,13-prostadiensäuremethylester

16. (5Z,13E)-(9R,11R,15S)-9-Chlor-3-oxa-15-cyclohexyl-11,15-dihydroxy-16,17,18,19,20-pentanor-5,13-prostadiensäureethylester

17. (5Z,13E)-(9R,11R,15S)-9-Chlor-3-oxa-15-cyclohexyl-11,15-dihydroxy-16,17,18,19,20-pentanor-5,13-prostadiensäureisopropylester

18. (5Z,13E)-(9R,11R,15S)-9-Chlor-15 cyclohexyl-11,15-dihydroxy-3-oxa-16,17,18,19,20-pentanor-5,13-prostadiensäure-tert.-butylester

19. Arzneimittel enthaltend eine oder mehrere Verbindungen aus den Ansprüchen 4 und 5 und übliche Hilfs- und Trägerstoffe.

## Claims

1. Pharmaceutical preparations comprising esters or amides of 9-chloroprostaglandins of formula (I) 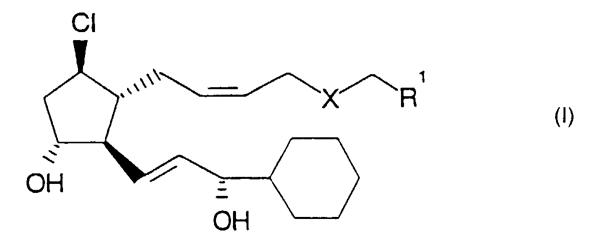 in which
X represents oxygen or CH₂, and
R¹ represents COOR², wherein R² is an optionally substituted C₁-C₁₀alkyi, C₃-C₁₀-cycloalkyl, C₆-C₁₀aryl or C₆-C₁₀ar(C₁-C₄)alkyl radical, or CONHR³, wherein R³ is an optionally substituted C₁-C₁₀alkyl radical,
for treating raised intraocular pressure.

2. Pharmaceutical preparations comprising 9-chloroprostaglandin esters or amides of the general formula (I) 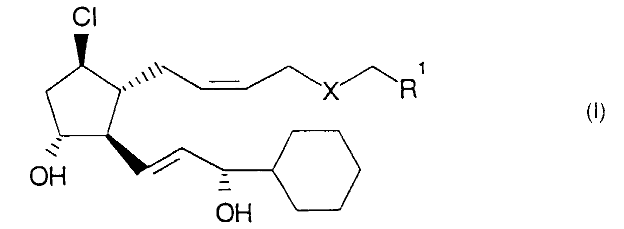 in which
X represents oxygen or CH₂, and
R¹ represents COOR², wherein R² is an optionally substituted phenacyl radical, or CONHR³, wherein R³ is an optionally substituted C₁-C₁₀alkyl radical,
for treating raised intraocular pressure and for cytoprotection, for healing ulcers, for inhibiting the secretion of gastric acid, for luteolysis, for reducing blood pressure or for inhibiting platelet aggregation.

3. Use of 9-chloroprostaglandin derivatives according to claim 1 and 2 in the preparation of medicaments for treating raised intraocular pressure.

4. 9-Chloroprostaglandin esters and amides of the general formula (I) 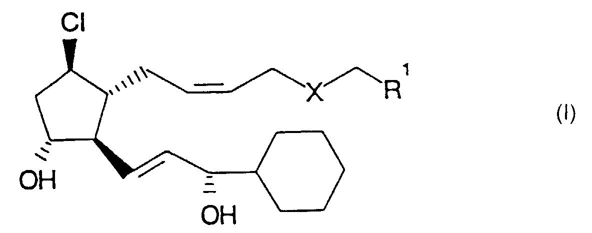 in which
X represents oxygen or CH₂, and
R¹ represents COOR², wherein R² is an optionally substituted phenacyl radical, or CONHR³, wherein R³ is an optionally substituted C₁-C₁₀alkyl radical.

5. Esters of 9-chloroprostaglandins of the general formula (I) 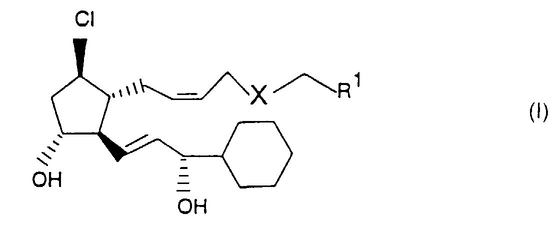 in which
X represents oxygen or CH₂, and
R¹ represents COOR², wherein R² is an optionally substituted C₂-C₃alkyl, cyclopentyl, cyclohexyl, phenyl or benzyl radical,
and their cyclodextrin clathrates.

6. Process for the preparation of 9-chloroprostaglandin esters and amides of formula (I), characterised in that a compound of formula (II) 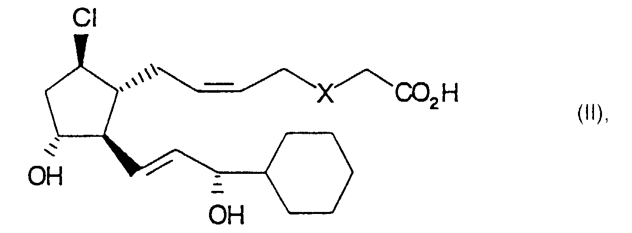 in which X is as defined above,
is reacted, with the addition of a suitable base, with an optionally substituted haloacetophenone derivative of the general formula (III) 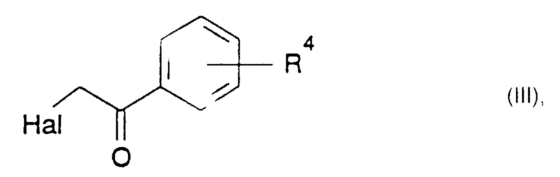 in which
R⁴ represents C₁-C₄alkyl, chlorine, bromine or iodine, and
Hal represents chlorine or bromine,
in a polar solvent to form compounds of the general formula (la) 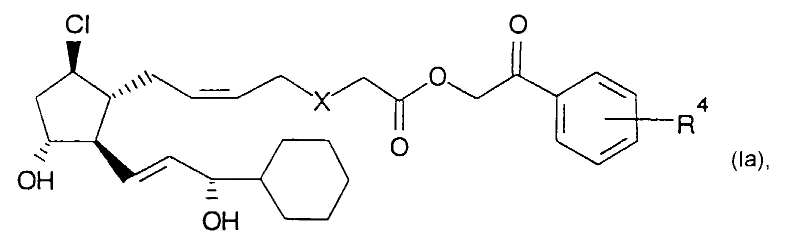 or
an alkyl ester of the general formula (Ib) 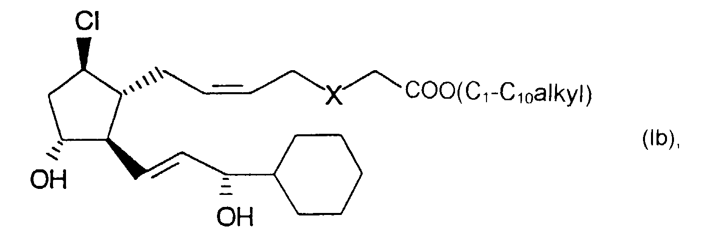 in which X is as defined above,
is reacted, without a solvent, with an amine of the general formula (IV)
H₂N-R³ (IV)
to form compounds of the general formula (Ic)

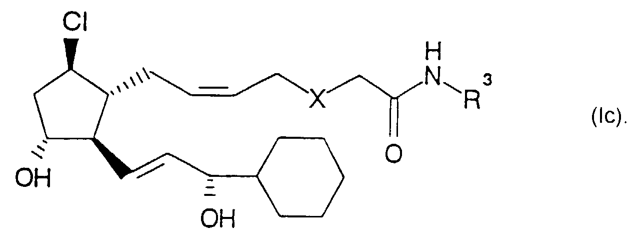

7. Process for the preparation of esters of 9-chloroprostaglandins of formula (I) in which
X represents oxygen or CH₂, and
R¹ represents COOR², wherein R² is an optionally substituted C₂-C₃alkyl, cyclopentyl, cyclohexyl, phenyl or benzyl radical,
characterised in that, in a manner known *per se,* a compound of formula (II) 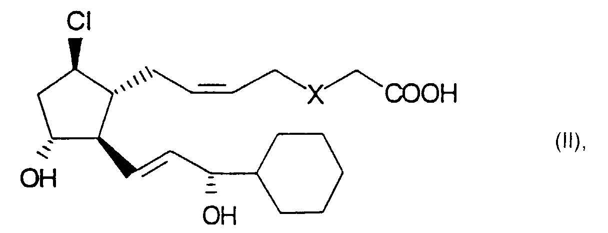 in which X is as defined above,
is either
A. reacted in the presence of a suitable base in a polar solvent with a compound of the general formula (III')
Hal-R² (III'),
in which R² is as defined above but is not phenyl and Hal represents chlorine, bromine or iodine, at temperatures of from -80°C to 100°C, and the resulting ester is isolated, or reacted in the presence of a suitable base in a polar solvent after activation with a carbodiimide such as, for example, dicyclohexylcarbodiimide with phenol at temperatures of from -30°C to 50°C, and the resulting ester is isolated.

8. (5Z,13E)-(9R,11R,15S)-9-Chloro-15-cyclohexyl-11,15-dihydroxy-16, 17,18,19,20-pentanor-5,13-prostadienoic acid phenacyl ester.

9. (5Z,13E)-(9R,11R,15S)-9-Chloro-15-cyclohexyl-11,15-dihydroxy-16, 17,18,19,20-pentanor-5,13-prostadienoicacid (3-hydroxypropyl)amide.

10. (5Z,13E)-(9R,11R,15S)-9-Chloro-3-oxa-15-cyclohexyl-11,15-dihydroxy-16,17,18,19,20-pentanor-5,13-prostadienoicacid phenacyl ester.

11. (5Z,13E)-(9R,11R,15S)-9-Chloro-3-oxa-15-cyclohexyl-11,15-dihydroxy-16,17,18,19,20-pentanor-5,13-prostadienoic acid (3-hydroxypropyl)amide.

12. (5Z,13E)-(9R,11R,15S)-9-Chloro-15-cyclohexyl-11,15-dihydroxy-16,17,18,19,20-pentanor-5,13-prostadienoic acid methyl ester.

13. (5Z,13E)-(9R,11R,15S)-9-Chloro-15-cyclohexyl-11,15-dihydroxy-16, 17,18,19,20-pentanor-5, 13-prostadienoicacid ethyl ester.

14. (5Z,13E)-(9R,11R,15S)-9-Chloro-15-cyclohexyl-11,15-dihydroxy-16,17,18,19,20-pentanor-5,13-prostadienoic acid isopropyl ester.

15. (5Z,13E)-(9R,11R,15S)-9-Chloro-3-oxa-15-cyclohexyl-11,15-dihydroxy-16,17,18,19,20-pentanor-5,13-prostadienoic acid methyl ester.

16. (5Z,13E)-(9R,11R,15S)-9-Chloro-3-oxa-15-cyclohexyl-11,15-dihydroxy-16,17,18,19,20-pentanor-5,13-prostadienoic acid ethyl ester.

17. (5Z,13E)-(9R,11R,15S)-9-Chloro-3-oxa-15-cyclohexyl-11,15-dihydroxy-16,17,18,19,20-pentanor-5,13-prostadienoic acid isopropyl ester.

18. (5Z,13E)-(9R,11R,15S)-9-Chloro-15-cyclohexyl-11,15-dihydroxy-3-oxa-16,17,18,19,20-pentanor-5,13-prostadienoic acid tert.-butyl ester.

19. Medicament comprising one or more compounds of claims 4 and 5 and customary excipients and carriers.

## Revendications

1. Préparations pharmaceutiques contenant des esters ou des amides des 9-chloro-prostaglandines de formule I 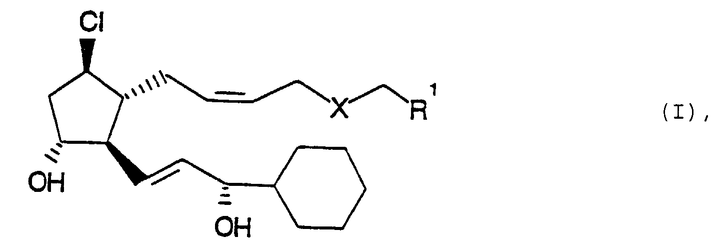 où
X représente un atome d'oxygène ou CH₂,
R¹ représente COOR²,
où R² représente des restes alkyle en C₁-C₁₀, cycloalkyle en C₃-C₁₀, aryle en C₆-C₁₀ ou (Ar en C₆-C₁₀)-(alkyle en C₁-C₄) éventuellement substitué ou CONHR³ avec R³ représentant un reste alkyle en C₁-C₁₀ éventuellement substitué,
pour le traitement de la tension intra-oculaire élevée.

2. Préparations pharmaceutiques contenant des esters ou des amides de la 9-chloro-prostaglandine de formule générale (I) 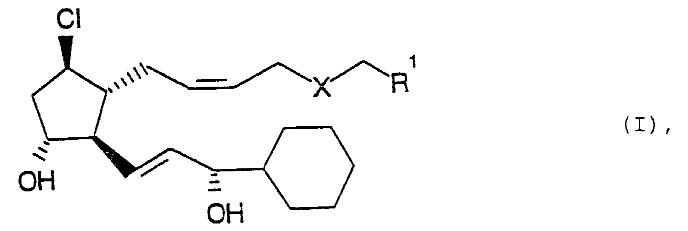 où
X représente un atome d'oxygène ou CH₂,
R¹ représente COOR²,
où R² représente un reste phénacyle éventuellement substitué,
ou CONHR³
avec R³ représentant un reste alkyle en C₁-C₁₀, éventuellement substitué,
pour le traitement de la tension intra-oculaire élevée et pour la cytoprotection, le traitement des ulcères, l'inhibition de la sécrétion d'acide gastrique, la lutéolyse, la diminution de la tension artérielle ou l'inhibition de l'agrégation plaquettaire.

3. Utilisation des dérivés de la 9-chloroprostaglandine selon la revendication 1 et 2 pour la préparation de médicaments pour le traitement de la tension intra-oculaire élevée.

4. Amides et esters de la 9-chloro-prostaglandine de formule générale I 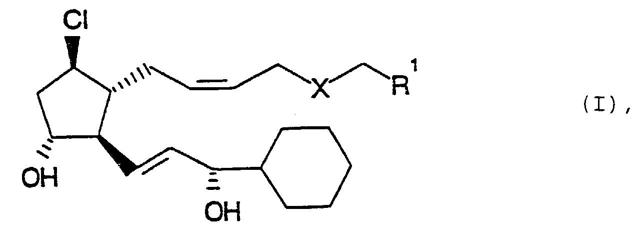 où
X représente un atome d'oxygène ou CH₂,
R¹ représente COOR²,
où R² représente un reste phénacyle éventuellement substitué,
ou CONHR³
avec R³ représentant un reste alkyle en C₁-C₁₀ éventuellement substitué,

5. Esters des 9-chloro-prostaglandines de formule générale I 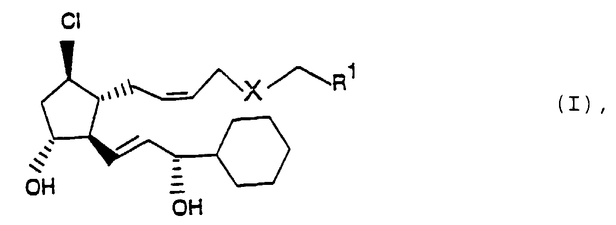 où
X représente un atome d'oxygène ou CH₂, et
R¹ représente COOR², R² représentant un reste alkyle en C₂-C₃, cyclopentyle, cyclohexyle, phényle ou benzyle éventuellement substitué et leurs clathrates de cyclodextrine.

6. Procédé pour la préparation d'esters et d'amides de la 9-chloro-prostaglandine de formule I, caractérisé en ce que l'on fait réagir
a) un composé de formule II 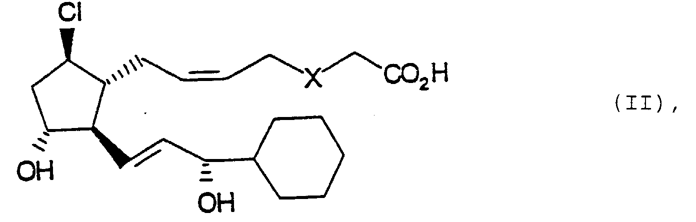 où
X a la signification donnée ci-dessus,
sous addition d'une base appropriée avec un dérivé halogéné d'acétophénone, éventuellement substitué, de formule générale III 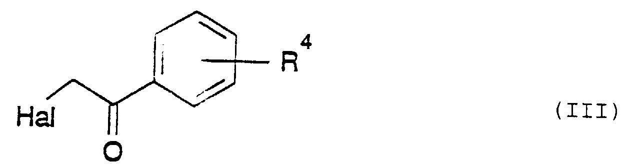 où
R⁴ représente un groupe alkyle en C₁-C₄, un atome de chlore, de brome ou d'iode, et
Hal représente chlore ou brome,
dans un solvant polaire pour les composés de formule (Ia), 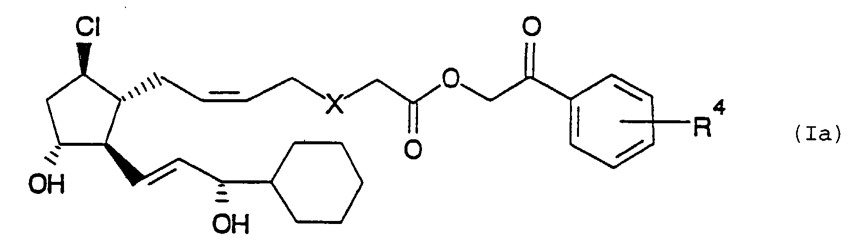 ou
un ester d'alkyle formule générale Ib 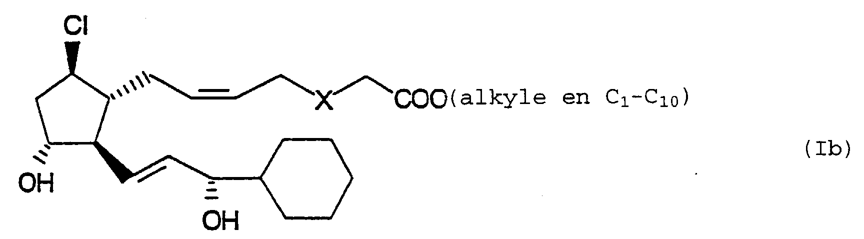 où
X a la signification donnée ci-dessus,
sans solvant, avec une amine de formule générale IV
H₂N-R³ (IV)
pour obtenir les composés de formule générale Ic

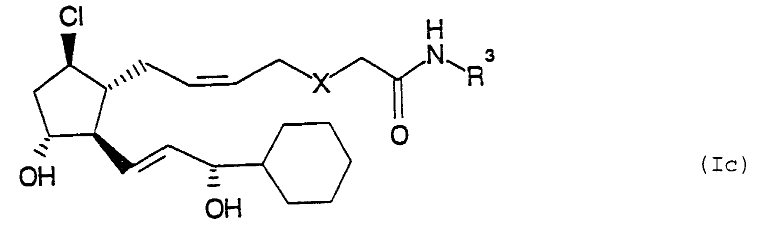

7. Procédé de préparation des esters des 9-chloroprostaglandines de formule I, où
X représente un atome d'oxygène ou CH₂, et
R¹ représente COOR², R² représentant un reste alkyle en C₂-C₃ cyclopentyle, cyclohexyle, phényle ou benzyle éventuellement substitué,
caractérisé en ce qu'on fait réagir de façon usuelle un composé de formule II 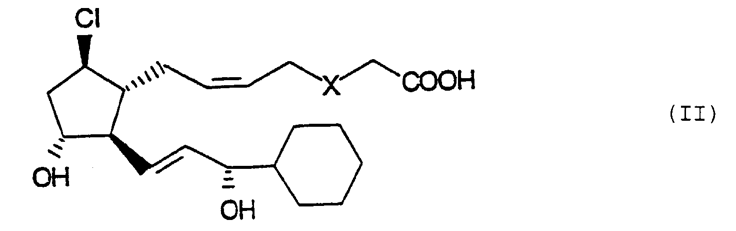 où
X a la signification donnée ci-dessus,
A. soit en présence d'une base appropriée dans un solvant polaire avec un composé de formule générale III'
Hal-R² (III')
où R² a la signification donnée ci-dessus, mais toutefois ne représente pas phényle, et Hal représente les atomes de chlore, de brome ou d'iode, à des températures comprises entre -80 °C et 100 °C, et on isole l'ester obtenu, soit on fait réagir en présence d'une base appropriée dans un solvant polaire, après activation par un carbodiimide, comme par exemple le dicyclohexylcarbodiimide avec du phénol, à une température comprise entre -30 °C et 50 °C, et on isole l'ester obtenu.

8. (5Z,13E)-(9R,11R,15S)-9-Chloro-15-cyclohexyl-11,15-dihydroxy-16,17,18,19,20-pentanor-5,13-prostadiénoate de phénacyle.

9. 3-Hydroxypropylamide d'acide (5Z,13E)-(9R,11R,15S)-9-chloro-15-cyclohexyl-11,15-dihydroxy-16,17,18,19,20-pentanor-5,13-prostadiénoïque.

10. (5Z,13E)-(9R,11R,15S)-9-Chloro-3-oxa-15-cyclohexyl-11,15-dihydroxy-16,17,18,19,20-pentanor-5,13-prostadiénoate de phénacyle.

11. 3-Hydroxypropylamide d'acide (5Z,13E)-(9R,11R,15S)-9-chloro-3-oxa-15-cyclohexyl-11,15-dihydroxy-16,17,18,19,20-pentanor-5,13-prostadiénoïque.

12. (5Z,13E)-(9R,11R,15S)-9-Chloro-15-cyclohexyl-11,15-dihydroxy-16,17,18,19,20-pentanor-5,13-prostadiénoatede méthyle.

13. (5Z,13E)-(9R,11R,15S)-9-Chloro-15-cyclohexyl-11,15-dihydroxy-16,17,18,19,20-pentanor-5,13-prostadiénoate d'éthyle.

14. (5Z,13E)-(9R,11R,15S)-9-Chloro-15-cyclohexyl-11,15-dihydroxy-16,17,18,19,20-pentanor-5,13-prostadiénate d'isopropyle.

15. (5Z,13E)-(9R,11R,15S)-9-Chloro-3-oxa-15-cyclohexyl-11,15-dihydroxy-16,17,18,19,20-pentanor-5,13-prostadiénoate de méthyle.

16. (5Z,13E)-(9R,11R,15S)-9-Chloro-3-oxa-15-cyclohexyl-11,15-dihydroxy-16,17,18,19,20-pentanor-5,13-prostadiénoate d'éthyle.

17. (5Z,13E)-(9R,11R,15S)-9-Chloro-3-oxa-15-cyclohexyl-11,15-dihydroxy-16,17,18,19,20-pentanor-5,13-prostadiénoate d'isopropyle.

18. (5Z,13E)-(9R,11R,15S)-9-Chloro-15-cyclohexyl-11,15-dihydroxy-3-oxa-16,17,18,19,20-pentanor-5,13-prostadiénoate de tertiobutyle.

19. Médicaments contenant un ou plusieurs composés selon les revendications 4 et 5 et des adjuvants et des matières de support usuels.
